# EUROPEAN PATENT APPLICATION

(11) **EP 4 732 776 A2**
(43) Date of publication of application: **29.04.2026**
(21) Application number: 26164623.6
(22) Date of filing: 24.08.2022
(51) Int. Cl.: A61B 6/00

(54) **SYSTEMS AND METHODS FOR MEASURING MOVEMENT OF A COMPRESSED BREAST**

(30) Priority: 25.08.2021 US 202163236762 P
(62) Divisional of application: 22769535.0
(71) Applicant: Hologic, Inc., Marlborough, MA 01752 (US)
(72) Inventor: REN, Baorui, Marlborough, 01752 (US); STEIN, Jay A., Marlborough, 01752 (US)
(74) Representative: Carpmaels & Ransford LLP

(57) **Abstract**

A method of imaging a breast compressed with a paddle including a foam compressive element. The method emits an x-ray energy from an x-ray source towards the breast and the foam compressive element and a marker disposed adjacent the foam compressive element. The x-ray energy is emitted over a predetermined time period. The marker includes a physical characteristic. The x-ray energy is detected at a detector disposed opposite the breast from the x-ray source. An image of the compressed breast and the marker is generated based on the detected x-ray energy. The marker in the generated image includes an image characteristic associated with the physical characteristic. The image characteristic is compared to the physical characteristic. A signal is sent when the image characteristic deviates from the physical characteristic by a threshold.

## Description

### Cross-Reference to Related Application

This application is being filed on August 24, 2022, as a PCT International Patent Application that claims priority to and the benefit of U.S. Provisional Application No. 63/236,762, filed on August 25, 2021, which application is hereby incorporated herein by reference in its entirety.

### Background

One known challenge in mammography and breast tomosynthesis is the discomfort the patient may feel when the breast is compressed, which must be done with sufficient force to immobilize the breast and, in some cases, spread out the breast tissues for x-ray imaging. Discomfort may potentially cause the patient to move, which negatively impacts image quality. Discomfort may also potentially dissuade patients from getting screened for breast cancer. Another known challenge is to ensure that the imaged field includes the desired amount of breast tissue. This discomfort has led to the development of compression paddles having flexible features, e.g., plastic sheets, flexible meshes, foam compressive materials, etc. These flexible features present unique challenges to properly image the breast. In existing imaging systems that utilize flat, rigid compression paddles, thickness of the compressed breast may be fairly easily determined by measuring or calculating a height of the compression paddle above a breast support platform. The use of flexible materials to stabilize a breast, however, make such simple calculations impossible.

### Summary

In one aspect, the technology relates to a method of imaging a breast compressed with a paddle including a foam compressive element, the method including: emitting an x-ray energy from an x-ray source towards the breast and the foam compressive element and a marker disposed adjacent the foam compressive element, wherein the x-ray energy is emitted over a predetermined time period, and wherein the marker includes a physical characteristic; detecting the x-ray energy at a detector disposed opposite the breast from the x-ray source; generating an image of the compressed breast and the marker based on the detected x-ray energy, wherein the marker in the generated image includes an image characteristic associated with the physical characteristic; comparing the image characteristic to the physical characteristic; and sending a signal when the image characteristic deviates from the physical characteristic by a threshold. In an example, the physical characteristic includes a physical dimension, and wherein the image characteristic depicts a movement of the marker during the emission of the x-ray energy. In another example, the physical dimension includes a marker width and wherein the marker width is greater than a width of a pixel on the detector. In yet another example, the marker is disposed on a surface of the foam compressive element that contacts the breast during the emission of the x-ray energy. In still another example, the marker is adhered to the breast.

In another example of the above aspect, the movement of the marker results from a movement of a portion of the breast during the emission of the x-ray energy. In an example, the portion of the breast is proximate the marker. In another example, the method further includes processing the image of the breast and the marker to generate an analysis image; and displaying the analysis image, wherein the marker is not visible in the analysis image. In yet another example, the physical characteristic includes a radiopacity, and wherein the image characteristic corresponds to an attenuation of the x-ray energy passing through the marker during the emission of the x-ray energy. In still another example, the marker includes a marker width and wherein the marker width is greater than a width of a pixel on the detector.

In another example of the above aspect, sending the signal includes displaying a notification.

In another aspect, the technology relates to an apparatus including: an x-ray source; an x-ray detector; a breast support platform for supporting a breast during an imaging procedure; a breast compression paddle movably disposed between the x-ray source and the x-ray detector, the breast compression paddle includes: a rigid substrate; a foam compressive element secured to the rigid substrate; and a marker disposed in contact with the foam compressive element: at least one processor; and a memory storing instructions that, when executed by the at least one processor, cause the apparatus to perform operations including: emitting an x-ray energy from the x-ray source and though the breast and the marker; receiving the emitted x-ray energy; generating an image from the received x-ray energy, wherein the image depicts the breast and the marker; analyzing the image to identify a movement of the marker; and sending a signal when the movement exceeds a threshold. In an example, analyzing the image includes identifying the marker in the image. In another example, the marker includes a physical characteristic and wherein analyzing the image includes comparing the physical characteristic of the marker to an imaged characteristic of the marker in the image. In yet another example, the physical characteristic includes a dimension of the marker. In still another example, the physical characteristic includes a radiopacity of the marker.

In another example of the above aspect, the operations further includes: processing the image of the breast and the marker to generate an analysis image; and displaying the analysis image, wherein the marker is not visible in the analysis image. In an example, sending the signal includes displaying a notification. In another example, the marker is disposed on a surface of the foam compressive element that is configured to contact the breast during the emission of the x-ray energy. In yet another example, the breast compression paddle includes the marker. In still another example, the marker is disposed on a sheet adhered to the breast.

In another example of the above aspect, the marker includes a plurality of markers arranged in at least one row. In an example, the at least one row includes a plurality of rows. In another example, the row is disposed substantially parallel to a chest wall of the patient. In yet another example, the row is disposed substantially orthogonal to a chest wall of the patient. In still another example, a first row of the plurality of rows is disposed substantially orthogonal to a second row of the plurality of rows.

### Brief Description of the Drawings

FIG. 1A is a schematic view of an exemplary imaging system.
FIG. 1B is a perspective view of the imaging system of FIG. 1A.
FIGS. 2A-2C are various views of a breast compression paddle having a foam compressive element.
FIGS. 3A and 3B depict partial side views of imaging systems having paddles utilizing foam compressive elements with markers thereon.
FIG. 3C depicts an adhesive sheet for securing markers to a breast.
FIGS. 4A and 4B depict an x-ray image and an enlarged partial x-ray image of a breast, respectively, depicting no detectable movement of the breast during an imaging procedure.
FIGS. 5A and 5B depict an x-ray image and an enlarged partial x-ray image of a breast, respectively, depicting detectable movement of the breast during an imaging procedure.
FIGS. 6A and 6B depicts an x-ray image and an enlarged partial x-ray image of a breast, respectively, depicting detectable movement of the breast during an imaging procedure.
FIGS. 7A-7C depict enlarged partial views of rows of imaged markers, indicating various degrees of movement thereof.
FIG. 8 depicts a method of imaging a breast compressed with paddle having a foam compressive element.
FIG. 9 depicts an example of a suitable operating environment in which one or more of the present examples can be implemented.

### Detailed Description

Breast compression paddles made entirely of rigid materials effectively compress a patient breast, as well as hold the breast still during imaging. While this is advantageous to obtain high-quality images, the discomfort associated with rigid paddles lead many women to avoid regular screening exams for breast cancer. Paddles utilizing foam compressive materials reduce patient discomfort during imaging procedures, which may persuade more women to have regular examinations, thus leading to early cancer diagnosis. However, while comfort is improved, the flexible nature of the thick foam compressive material may allow for movement of the breast during imaging procedures (e.g., if the patient pulls away from the breast imaging system, inadvertently or deliberately). If this occurs, the resulting image may be less diagnostically-relevant than desired. Thus, while paddles utilizing foam compressive elements are more comfortable, such paddles introduce myriad challenges to how a breast imaging system (e.g., a mammography, tomosynthesis, or combination mammography/tomosynthesis system) takes exposures and produces images of diagnostically-relevant quality.

The technologies described herein provide devices and methods to accurately detect movement of a breast under a foam compressive element of a compression or immobilization paddle. The technology contemplates markers on areas of the foam compressive element that may move when subject to an applied force. For example, if a patient breast is compressed by a paddle having a foam compressive element, movement of the patient (e.g., pulling away from the imaging system) would be translated to portions of the foam compressive element proximate the breast, but not to portions of the rigid substrate, which are less susceptible to movement and located far from the breast. The amount of movement of the marker(s) may be detected in a variety of ways, for example, via an analysis of the resulting image (and the imaged marker therein) or via an analysis of discrete pixels of an x-ray detector. If the detected movement exceeds a threshold (e.g., so as to no longer be indicative of a diagnostically-relevant image), a notification may be triggered. This notification may require the breast imaging procedure to be performed again, which may take place immediately, while the patient is still at the medical clinic (and even under compression). This helps save time by ensuring the image is actually of sufficient quality, before being the time-consuming review and analysis by a radiologist. Thus, these technologies are critical to make viable paddles utilizing a foam compressive material.

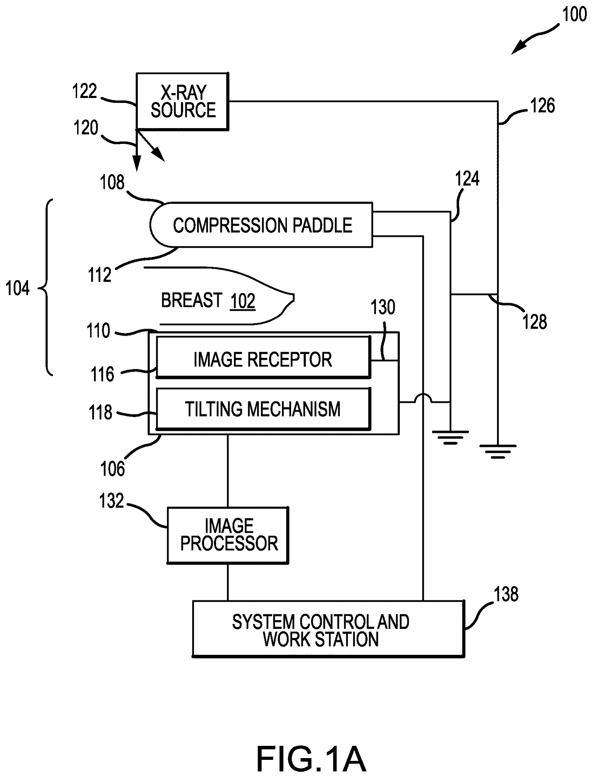

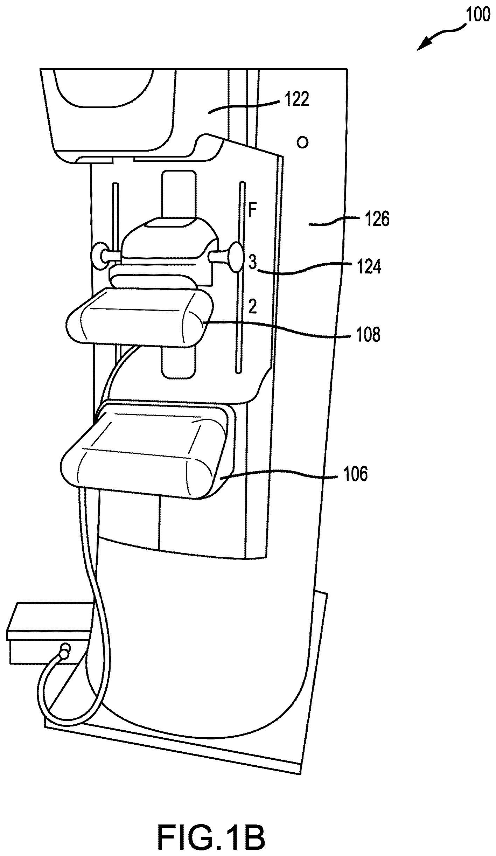

FIG. 1A is a schematic view of an exemplary imaging system 100. FIG. 1B is a perspective view of the imaging system 100. Referring concurrently to FIGS. 1A and 1B, the imaging system 100 immobilizes a patient's breast 102 for x-ray imaging (either or both of mammography and tomosynthesis) via a breast compression immobilizer unit 104 that includes a static breast support platform 106 and a moveable compression paddle 108. The breast support platform 106 and the compression paddle 108 each have a compression surface 110 and 112, respectively, that move towards each other to compress and immobilize the breast 102. In known systems, the compression surface 110, 112 is exposed so as to directly contact the breast 102. The platform 106 also houses an image receptor 116 and, optionally, a tilting mechanism 118, and optionally an anti-scatter grid. The immobilizer unit 104 is in a path of an imaging beam 120 emanating from x-ray source 122, such that the beam 120 impinges on the image receptor 116.

The immobilizer unit 104 is supported on a first support arm 124 and the x-ray source 122 is supported on a second support arm 126. For mammography, support arms 124 and 126 can rotate as a unit about an axis 128 between different imaging orientations such as CC and MLO, so that the system 100 can take a mammogram projection image at each orientation. In operation, the image receptor 116 remains in place relative to the platform 106 while an image is taken. The immobilizer unit 104 releases the breast 102 for movement of arms 124, 126 to a different imaging orientation. For tomosynthesis, the support arm 124 stays in place, with the breast 102 immobilized and remaining in place, while at least the second support arm 126 rotates the x-ray source 122 relative to the immobilizer unit 104 and the compressed breast 102 about the axis 128. The system 100 takes plural tomosynthesis projection images of the breast 102 at respective angles of the beam 120 relative to the breast 102.

Concurrently and optionally, the image receptor 116 may be tilted relative to the breast support platform 106 and in sync with the rotation of the second support arm 126. The tilting can be through the same angle as the rotation of the x-ray source 122, but may also be through a different angle selected such that the beam 120 remains substantially in the same position on the image receptor 116 for each of the plural images. The tilting can be about an axis 130, which can but need not be in the image plane of the image receptor 116. The tilting mechanism 118 that is coupled to the image receptor 116 can drive the image receptor 116 in a tilting motion. For tomosynthesis imaging and/or CT imaging, the breast support platform 106 can be horizontal or can be at an angle to the horizontal, e.g., at an orientation similar to that for conventional MLO imaging in mammography. The system 100 can be solely a mammography system, a CT system, or solely a tomosynthesis system, or a "combo" system that can perform multiple forms of imaging. An example of such a combo system has been offered by the assignee hereof under the trade name Selenia Dimensions.

When the system is operated, the image receptor 116 produces imaging information in response to illumination by the imaging beam 120, and supplies it to an image processor 132 for processing and generating breast x-ray images. A system control and work station unit 138 including software controls the operation of the system and interacts with the operator to receive commands and deliver information including processed-ray images.

One challenge with the imaging system 100 is how to immobilize and compress the breast 102 for the desired or required imaging. A health professional, typically an x-ray technologist, generally adjusts the breast 102 within the immobilizer unit 104 while pulling tissue towards imaging area and moving the compression paddle 108 toward the breast support platform 106 to immobilize the breast 102 and keep it in place, with as much of the breast tissue as practicable being between the compression surfaces 110, 112. This can cause discomfort to the patient.

To improve comfort for the patient, compression paddles utilizing foam compressive elements have been developed. Compression paddles utilizing foam compressive elements are described generally in PCT International Patent Application Nos. PCT/US2019/033998, PCT/US2019/034001, and PCT/US2019/034010, all filed May 24, 2019, the disclosures of which are hereby incorporated by reference herein in their entireties. Such paddles stabilize and compress slightly the breast, while reducing discomfort associated with compression paddles having only rigid compressive surfaces.

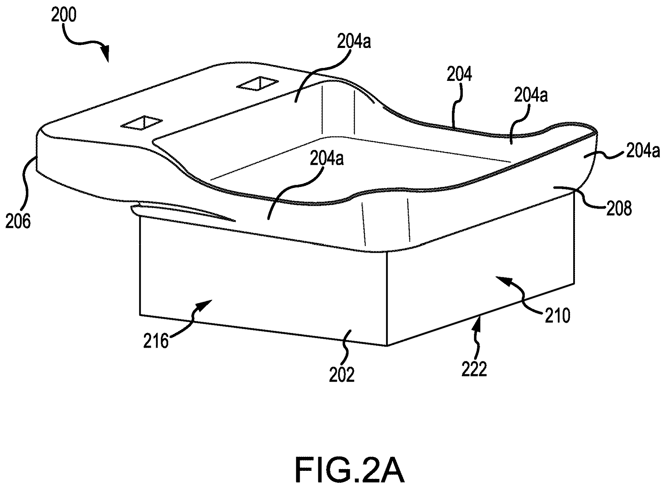

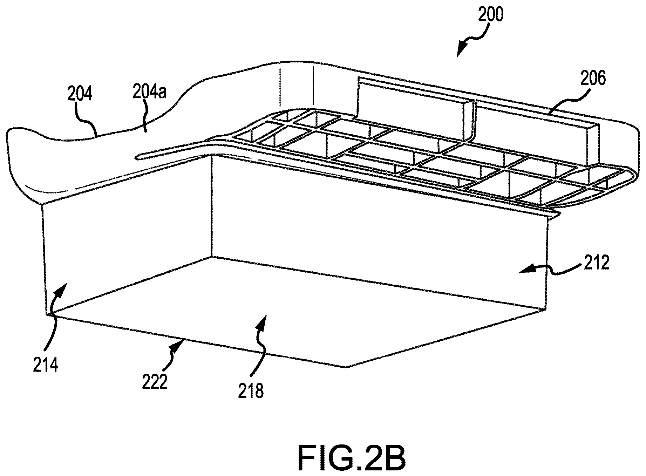

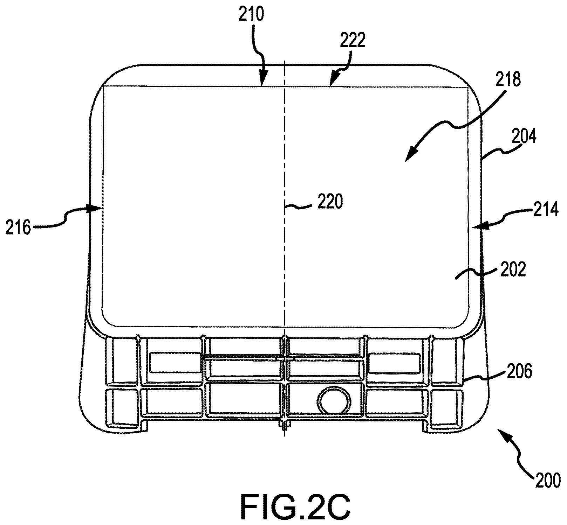

FIGS. 2A-2C are various views of one example of a breast compression paddle 200 having a foam compressive element 202 secured to a rigid substrate 204. FIGS. 2A-2C are described concurrently. The paddle 200 includes a bracket portion 206, generally integral with the substrate 204 for connecting the paddle to compression arm of an imaging system. The paddle 200 also includes a leading face 208, opposite the bracket portion 206, which is disposed proximate a chest wall of a patient during compression and imaging procedures. In examples, the substrate may be rigid. As used herein, the term "rigid" does not imply that the substrate 204 is free from bending during compression of a breast, rather that the substrate 204 displays greater resistance to bending or deformation than the foam compressive element 202 secured to a bottom of the substrate 204. Raised walls 204a provide additional rigidity.

The foam compressive element 202 may be secured to a bottom surface of the substrate 204 with a chemical adhesive. In other example, an upper surface of the compressive element may be a rigid plastic or other material to which the foam compressive element 202 is secured. A plurality of bolts, hooks, or other mechanical fasteners (not shown) may be used to connect this rigid plastic to the rigid substrate 204 of the paddle 200. If such mechanical fasteners are used, it may be desirable to dispose said fasteners away from areas of the foam compressive element 202 that are expected to compress against a breast, so as to avoid pressure points and resulting discomfort associated therewith, as well as to prevent artifacts from appearing in any resulting x-ray images.

The foam compressive element 202 includes a number of edge surfaces. A leading edge surface 210 is disposed proximate the leading face 208 of the substrate 204 so as to be disposed proximate the chest wall of a patient during compression and imaging procedures. A trailing edge surface 212 is disposed opposite the leading edge surface 210, proximate the bracket portion 206. Lateral edge surfaces 214, 216 are also depicted. In general, these lateral edge surfaces 214, 216 may be depicted as inner or outer lateral edge surfaces, consistent with terminology typically used to describe inner and outer sides of the breast. Of course, a person of skill in the art will recognize that the same compression paddle 200 may be used to compress either breast, one at a time, which would effectively change the application of the terms "inner" and "outer" to the lateral edge surfaces of the foam compressive element 202. Further, a mid-plane 220 is disposed between the lateral edge surfaces 214, 216, at an approximate midpoint thereof. The mid-plane 220 is disposed substantially orthogonal to a compressive surface 218 that is disposed on an underside of the foam compressive element 202. Portions of the compressive surface 218 will contact the breast during compression. In another example, the foam compressive element 202 may be covered with a biocompatible cover, which may protect the foam compressive element 202 from absorbing bodily fluids. In examples, the cover may be disposable or cleanable. To improve the patient experience, the cover may be manufactured of a soft material where it contacts the patient. To prevent fluid transfer into the foam compressive element 202, an opposite plastic side may contact the foam compressive element 202. An interface 222 is located where the compressive surface 218 meets the leading edge surface 210. The shape of the interface 222 during compression aids in defining the foam compressive element 202 and the function thereof.

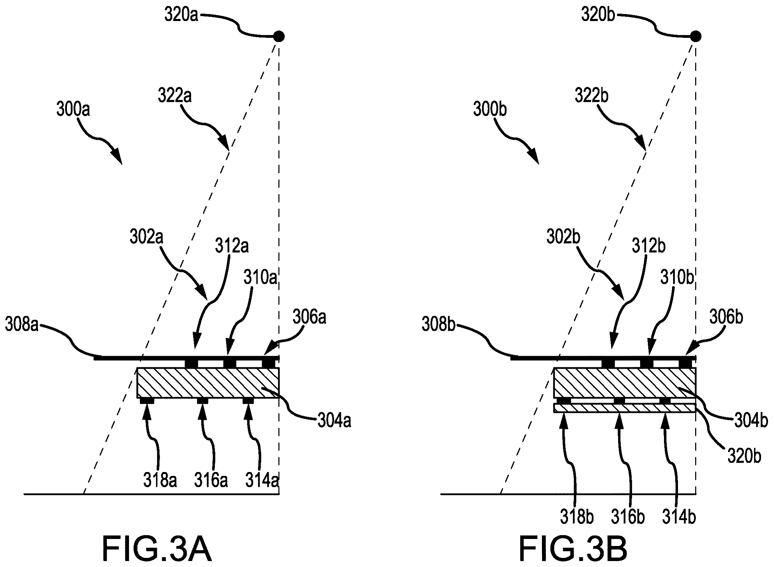

FIGS. 3A and 3B depict partial side views of imaging systems 300 having paddles 302 utilizing foam compressive elements 304 with a plurality of markers thereon. Unless otherwise noted, suffixes of reference numerals depicted in FIGS. 3A and 3B correspond to the specific features described without suffixes in the description of those figures. The plurality of markers 306, 310, 312, 314, 316, 318, arranged in multiple rows proximate top and bottom surfaces of the foam compressive element 304 (as described in more detail herein), allow for the detection of movement of the compressed breast. As noted above, markers located in or on the foam compressive element 304 proximate the breast (e.g., markers 314, 316, 318) will more likely move as a result of breast movement. As such, markers 314, 316, 318 disposed on the foam compressive element are more relevant to the technologies described herein, but markers on the rigid substrate 308 (e.g., markers 306, 310, 312) may also be included for various purposes. For example, while detected movement of a marker 314, 316, 318 located proximate the breast (e.g., on or in the foam compressive element) might be in excess of a certain threshold to trigger a notification signal, detected movement of a marker 306, 310, 312 distal from the breast (e.g., at the rigid substrate) may trigger a notification signal in excess of a much lower threshold. This is because detected movement of the rigid paddle is so unlikely that any movement whatsoever may be indicative of some type of problem with the paddle or compression system, significant movement of the patient, or some other error or issue. For clarity, however, for the purposes of this application, the motion detection technologies will be described in the context of movement of the markers closer to the breast, such as markers 314, 316, 318.

Each paddle 302 includes a rigid substrate 308 and a foam compressive element 304 secured at or near a bottom surface thereof. In the figures, the rigid substrate 308 is depicted spaced apart from the foam compressive element 304, but this is for illustrative purposes only. Secured between the rigid substrate 308 and the foam compressive element 304 are a plurality of top markers 306, 310, 312 arranged in rows parallel to the chest wall. The first row of top markers 306 is depicted closest to the chest wall, for example, proximate a front surface of the rigid substrate 308 and the foam compressive element 304. A second row of top markers 310 and a third row of top markers 312 are disposed known distances from the first row 306. The top markers 306, 310, 312 may be secured to the rigid substrate 308 or the foam compressive element 304 and may be disposed between those elements. In another example, the top markers 306, 310, 312 may be disposed on an upper surface of the rigid substrate 308. Further, a greater or lesser number of marker rows may be utilized.

Secured to or near a bottom surface of the foam compressive element 304 are a plurality of bottom markers 314, 316, 318 arranged in rows parallel to the chest wall. The first row of bottom markers 314 are depicted closest to the chest wall. A second row of bottom markers 316 is disposed a distance from the first row 314, while a third row of bottom markers 318 are disposed another distance from the second row 316. Other numbers of rows are contemplated. The bottom markers 314, 316, 318 may be exposed at the bottom surface of the foam compressive element 304a, as depicted in FIG. 3A, or may be covered by a thin foam cover 320b, as depicted in FIG. 3B. The thin foam cover 320b may be formed from the same material, or a different material as the foam compressive element 304, and may be about 5%, about 7%, about 10%, about 12%, about 15%, or about 17% of the thickness of the foam compressive element 304. The distance between the various rows of top markers 306, 310, 312 and bottom markers 314, 316, 318 may be as required or desired for a particular application. In general, the separation distances should allow each of the rows of markers 306, 310, 312, 314, 316, 318 to be visible in a resulting image generated by x-ray emission from an x-ray tube (the focal spot thereof depicted at 320). Thus, given the angle of x-ray energy 322, the second row of bottom markers 316 would still be visible in the resulting image, due to the distance between the second row of top markers 310 and the third row of top markers 312. The separation distances may be determined based at least in part on the thickness of the foam compressive element 304, the length of the individual markers in each row of markers 306, 310, 312, 314, 316, 318, the anticipated distance between the focal spot 322 and the markers 306, 310, 312, 314, 316, 318, and other factors.

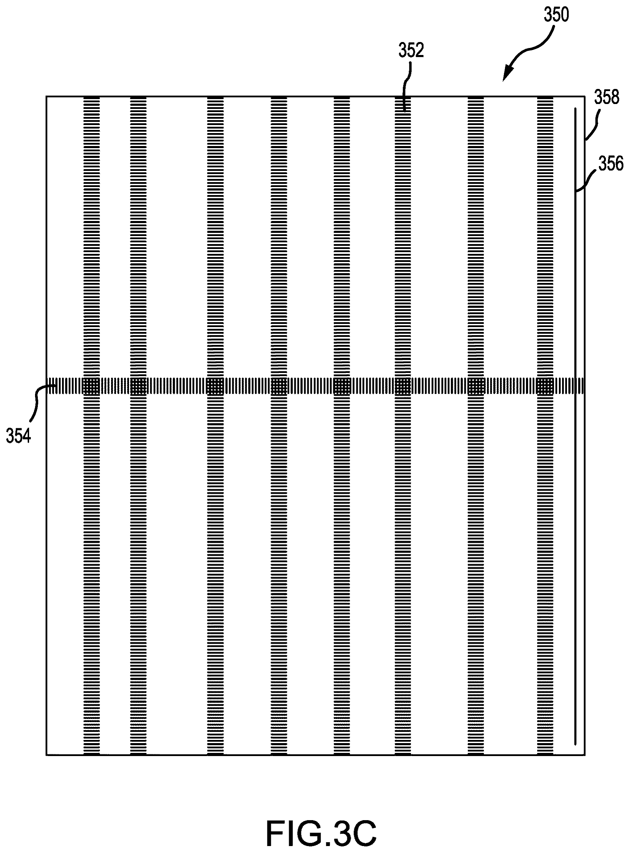

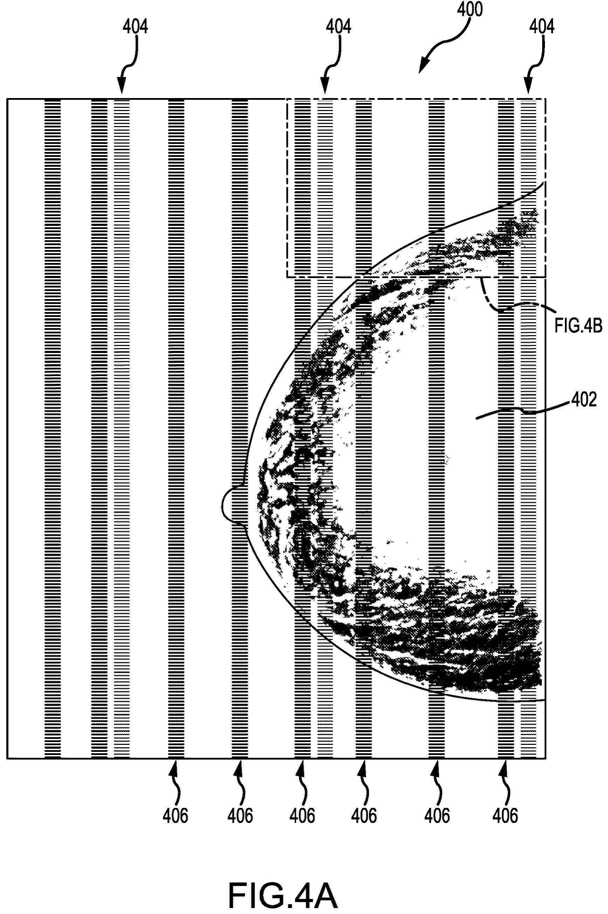

An alternative marker system is depicted in FIG. 3C, which depicts an adhesive sheet 350 including a plurality of markers 352, 354. The sheet 350 may include, on one side, an adhesive that may enable the sheet 350 to be adhered temporarily to a breast for imaging. A contact paper (not shown) may cover the adhesive during storage and may be removed prior to adhesion of the sheet 350 to the breast. The sheet 350 may have a defined (e.g., marked with an indicia 356) edge 358 that is intended to be disposed adjacent to a chest wall of a patient during adhesion. This enables the technologist imaging the patient to easily apply the sheet 350 prior to imaging. The plurality of markers 352, 254 may be arranged in a plurality of rows, e.g., as depicted above in FIGS. 3A-3B and below in FIGS. 4A-4B, et seq. In the depicted example, a plurality of rows of markers 352 are arranged so as to be substantially parallel to the edge 358 that is adjacent the chest wall. A single row of markers 354 are arranged so as to be substantially orthogonal to the edge 358. Multiple rows of markers 354 may also be utilized. By using one or more rows of markers 354 arranged orthogonally to the chest wall, movement substantially orthogonal to the chest wall (e.g., resulting from the patient pulling away from compression) may be more easily detected. These rows of markers 354 arranged orthogonally to the chest wall may also be utilized in the configurations depicted in the following FIGS., but are not depicted there for clarity.

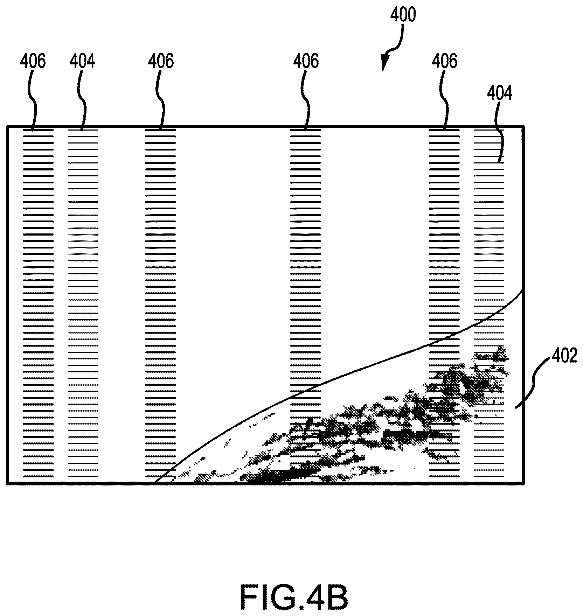

FIGS. 4A and 4B depict an x-ray image 400 and an enlarged partial x-ray image 400 of a breast 402, respectively, depicting no detectable movement of the breast 402 during an imaging procedure. The image 400 depicts the breast 402, a plurality of rows of top markers 404 and a plurality of rows of bottom markers 406. Rows of top markers 404 are those markers that are installed on a rigid portion of a compression paddle and are distal the breast 402, e.g., between a foam compressive element and a rigid substrate of the compression paddle. Bottom markers 406 are those installed on or in the foam compressive element, proximate the breast 402. In an alternative example, bottom rows 406 of markers may be those disposed on an adhesive sheet, such as depicted in FIG. 3C. For clarity, however, the markers 406 in FIGS. 4A and 4B are described in the context of being on or in the foam compressive element 402 of a compression paddle. The visibility of such markers may be enhanced or reduced with known image processing techniques. In each row of both the top markers 404 and bottom markers 406, discrete markers may be the same width and spaced at regular separation intervals. For example, rows of both top markers 404 and rows of bottom markers 406 may include individual markers of 250 µm wide, and having a separation distance of 250 µm. In other examples, markers of 300 µm wide, 300 µm separation distance are also contemplated. Other examples include markers of 200 µm wide, 200 µm separation distance; markers of 350 µm wide, 350 µm separation distance; and other configurations. Markers may also have a width greater than or less than a separation distance between adjacent markers. In general, the separation distance provides space around discrete markers to enable detection of movement of the discrete markers and, by extension, the breast itself. In the image 400, both the row of top markers 404 and the row of bottom markers 406 present defined separation between adjacent markers of each row 404, 406. As such, in FIG. 4A, the consistency of marker width and separation distance indicates that the marker (and therefore the breast) did not move during emission of the x-ray energy.

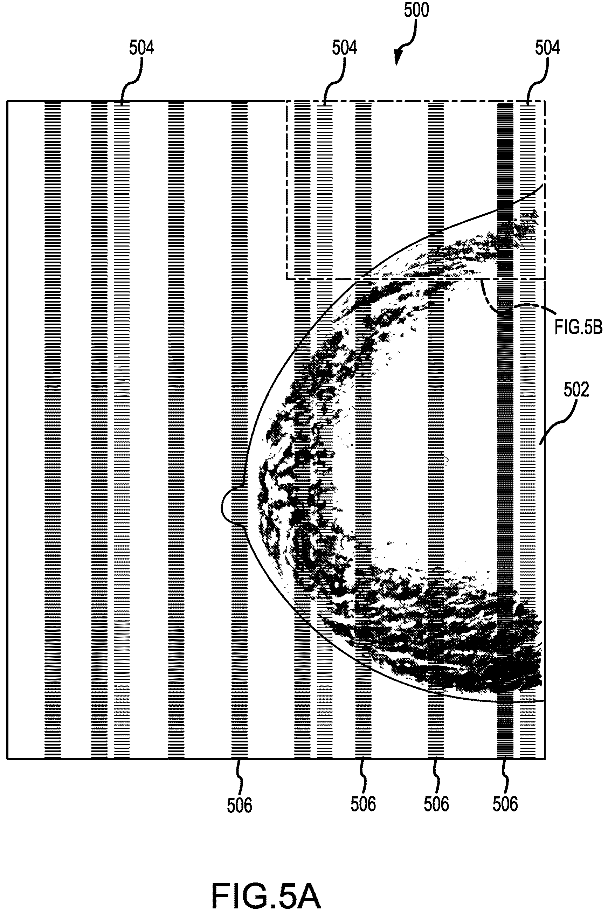

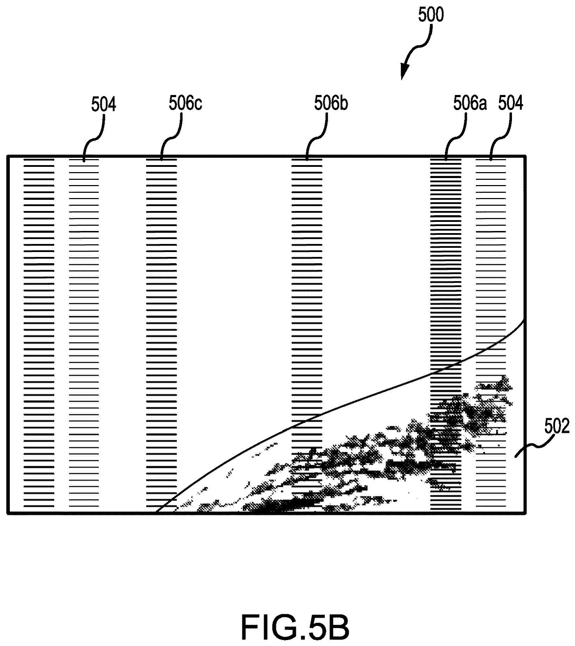

FIGS. 5A and 5B depict an x-ray image 500 and an enlarged partial x-ray image 500 of a breast 502, respectively, depicting movement of the breast 502 during an imaging procedure. The detectable movement is depicted by a change in appearance of one or more of the rows of bottom markers 506a, 506b, and 506c. Rows of top markers 504, given their location proximate the rigid substrate, generally do not move. Thus, the discrete markers of the rows of top markers 504 appear at a consistent width and spacing. With regard to the rows of bottom markers 506, however, movement is depicted by the distortion of the imaged individual markers in the row. For example, in the row of bottom markers 506a, closest to the chest wall, individual markers have moved. Over the period of time of emission of the x-ray, this movement is captured as an apparent enlarging of the width of each discrete marker in the row of bottom markers 506a, and a corresponding decrease in separation distance between adjacent markers. For the purposes of explanation, assuming markers having a physical width and separation distance of 250 µm for each, in the image, the imaged width may be about 500 µm, resulting in an imaged separation distance of about 100 µm. This indicates movement of the markers. Since the foam compressive element is in contact with the breast proximate this row of bottom markers 506a, the movement of the markers corresponds to that of the breast. The other rows of bottom markers (e.g., rows 506b, 506c, 506d, etc.) do not reflect any movement thereof.

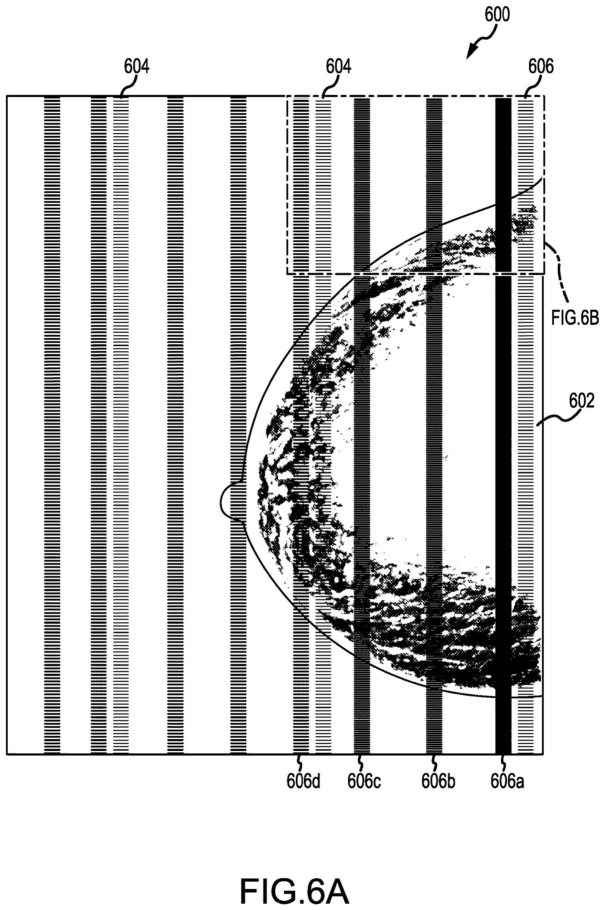

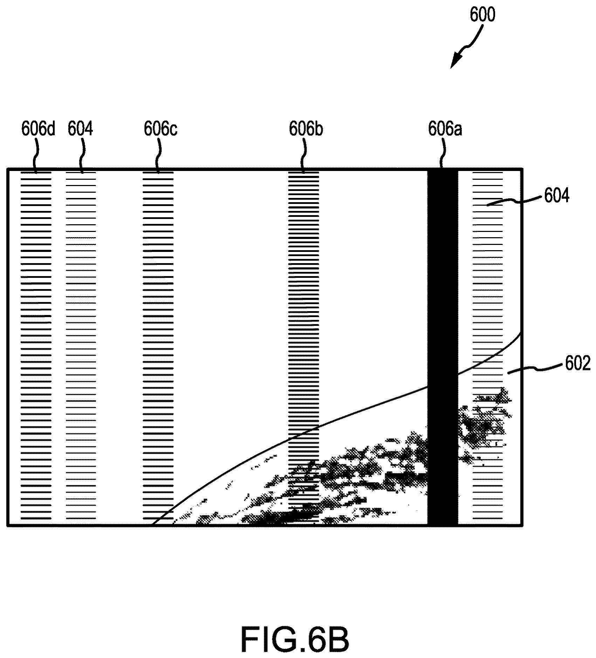

FIGS. 6A and 6B depict an x-ray image 600 and an enlarged partial x-ray image 600 of a breast 602, respectively, depicting movement of the breast 602 during an imaging procedure. The detectable movement is depicted by a change in appearance of one or more of the rows of bottom markers 606a, 606b, and 606c. Rows of top markers 604, given their location proximate the rigid substrate, generally do not move. Thus, the discrete markers of the rows of top markers 604 appear at a consistent width and spacing. With regard to the rows of bottom markers 606, however, movement is depicted by the distortion of the imaged individual markers in the row. For example, in the row of bottom markers 606a, closest to the chest wall, individual markers have moved, causing a complete "blur" or "smear" of the entire row of bottom markers 606a. Using markers having widths of 250 µm, with 250 µm separation distance, it is clear that the row of bottom markers 606a has moved a full 250 µm, thus eliminating any distinction between discrete markers in that row 606a. Since the foam compressive element is in contact with the breast proximate this row of bottom markers 606a, the movement of the markers corresponds to that of the breast.

The row of bottom markers 606b also displays some distortion of individual markers, as indicative of wider markers and, by extension, reduced separation distance therebetween. This distortion of markers is indicative of some movement of the breast, but not as much as that of the portion of the breast proximal the first row of bottom markers 606a. This is because different portions of the breast may move differently under a foam compressive element. The row of bottom markers 606c displays no apparent distortion of individual markers, thus indicating no movement of the breast proximate that location. Thus, with these rows of bottom markers 606a, 606b, 606c, it is clear that significant movement of the breast occurs proximate row 606a, some movement proximate row 606b, and no detectable movement proximate row 606c.

The rows of markers depicted in FIGS. 4A-6B are disposed substantially parallel to the chest wall, with the individual markers extending substantially orthogonal to the chest wall. Thus, such markers are configured to more easily indicate movement parallel to the chest wall. The technologies described herein may also be utilized to detect movement substantially orthogonal to the chest wall (e.g., in instances where a patient pulls away from the compression system). In such a configuration, it may be desirable to arrange rows of markers extending in a direction substantially orthogonal to the chest wall, with individual markers being disposed substantially parallel thereto.

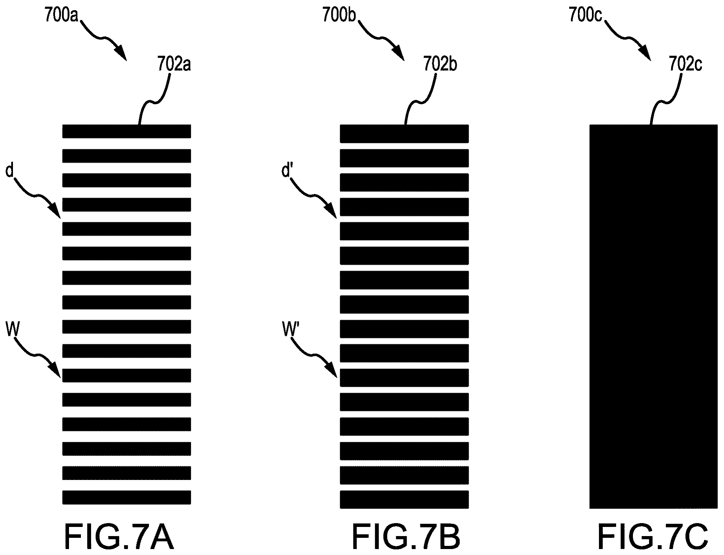

As described herein, each marker has a "physical" characteristic and an "imaged" characteristic. Physical characteristics are characteristics of the marker in the physical world, e.g., length, width, separation distance between adjacent markers, radiopacity, and so on. Imaged characteristics are characteristics of the markers in an image. For example, in an image, the imaged width of a marker is depicted. Typically, the imaged width (or other imaged dimensions, such as length and separation distance), are related to the associated physical characteristic, but likely differ therefrom in the image. For example, the marker may appear larger or smaller in the image, depending on distance between the marker and detector, magnification, or other factors. Similarly, the radiopacity of a physical marker will dictate, at least in part, the appearance of an artifact of the marker in the image. With these terms in mind, FIGS 7A-7C are now described.

FIGS. 7A-7C depict enlarged partial views of rows 700 of markers 702, indicating various degrees of movement thereof. Unless otherwise noted, suffixes of reference numerals depicted in FIGS. 7A-7C correspond to the specific features described without suffixes in the description of those figures. The scale of each of FIGS. 7A-7C is exaggerated for illustrative purposes. FIG. 7A depicts a condition where no movement of markers 702 is present in a resulting x-ray image. The row of markers 700a include a plurality of discrete markers 702a that, in the image, have a defined imaged width W and are separated by an imaged separation distance d. If the physical width and separation distance of the actual markers on a compressive foam element of a compression paddle are known, the imaged width W and imaged separation distance d can be compared. Methods of making such comparisons are described below. Since the imaged width W and imaged separation distance d of the markers 702a correspond to the physical width and separation distance (factoring in magnification, as known in the art), it can be concluded that no detectable movement of the markers 702a (and therefore adjacent portions of the breast) has occurred.

FIG. 7B depicts a marker condition where some movement is detected in an x-ray image. The row of markers 700b includes a plurality of discrete markers 702b, that, in the image, have a larger imaged width W' and are separated by a smaller imaged distance d', both of which do not correspond to the physical width and physical separation distance of the actual markers on the compressive foam element of the compression paddle. Thus, by comparing the imaged width W' and/or the imaged separation distance d' to the known physical width and physical separation distance, the amount of movement may be determined. Methods of comparisons are described below. The imaged width W' and imaged separation distance d' result from the changing position of the markers 702b over the time of emission of the x-ray energy due to movement thereof. Apparent movement of the markers 702b, however, is captured in the image as the larger imaged width W' and smaller imaged separation distance d'. The larger imaged width W' and smaller imaged separation distance d' may be calculated to determine the total amount of movement, again considering magnification and other known factors.

FIG. 7C depicts a marker condition where significant movement is detected in an x-ray image. The imaged row of markers 700c appears in the image as a single marker 702c. Thus, it can be concluded that the imaged physical markers have moved so much so as to merge into a single, large, smeared artifact in the image. Neither marker width nor separation distance is discernible, indicating that the markers have moved by a distance at least equal to the physical separation distance of the markers on the foam compressive element. The change in imaged artifact results from the changing position of the markers 702c over the time of emission of the x-ray energy. Such a significant movement may cause the imaging system to issue a notification that the breast moved so much during imaging so the resulting image is not considered diagnostically relevant. Movement of a lesser magnitude than that depicted in FIG. 7C may also trigger the notification.

The imaged marker width and/or imaged separation distance may be determined in several ways. Each marker located on the compression paddle (either the top markers or bottom markers, as described above), may be characterized by a physical characteristic as noted above. Each marker is defined by dimensional information (e.g., length, width) and radiopacity. Changes to these physical characteristics are reflected in the image when the marker moves during imaging. In one example, the imaged dimensions of the markers may be analyzed to identify any changes that may be have occurred due to movement. These are most easily contemplated in the context of FIGS. 7A-7C above by changes in the width and separation distance, which may be measured by image recognition techniques in the resultant x-ray images, or may be measured on a per-pixel basis at the detector. In the latter example, the pixel width of a marker at a particular distance from the detector and at a particular magnification is known. For purposes of illustration, that marker may be three pixels wide. If, however, the imaged width is five pixels, it can be concluded that the marker moved the equivalent of two pixels along the marker width during x-ray emission. As such, it is often desirable that the markers are wider than the dimensions of a pixel.

In another example, the x-ray energy received at each pixel of the detector may also be measured to detect x-ray absorption at each pixel. Referring again to FIG. 7A, which depicts no movement of the markers, the amount of x-ray energy detected at each pixel corresponding to a location of a marker is unchanged during the x-ray emission. This enables the imaging system to determine that no marker movement (and therefore, no breast movement) has occurred. In FIG. 7B, however, each pixel located at each imaged marker receives a comparatively greater amount of x-ray energy over the time of emission of the x-ray energy, due to the movement of each marker. In other words, for a given pixel located below a marker, the pixel will receive an amount of x-ray energy reduced by the presence of the marker (due to the marker's radiopacity). If that marker moves, however, the x-ray energy will be unimpeded to the same pixel for at least a period of time, thus causing the total amount of x-ray energy detected at each pixel to be different than if the marker had remained stationary above the pixel for the entire emission. In FIG. 7C, the amount of x-ray energy at an even greater number of pixels is altered during x-ray emission, as the physical marker moves an even greater distance during imaging procedures.

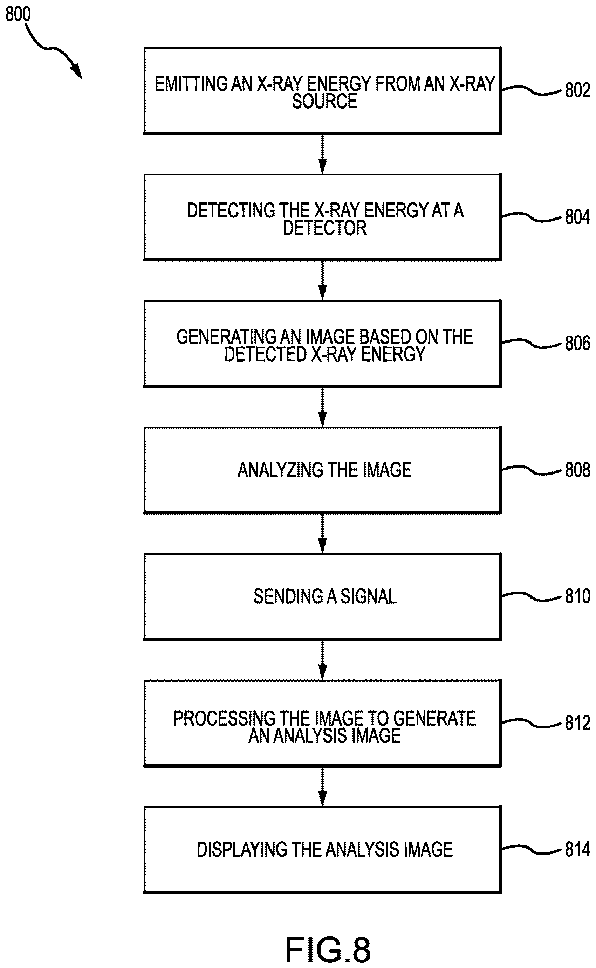

FIG. 8 depicts a method 800 of imaging a breast compressed with a paddle having a foam compressive element. The method 800 begins with operation 802, emitting an x-ray energy from an x-ray source. The x-ray energy is emitted towards a breast that is immobilized or compressed by the paddle and the foam compressive element. A plurality of markers are disposed on or in either or both of a rigid substrate of the paddle and the foam compressive element. The x-ray energy is emitted over a predetermined period of time, e.g., less than about 0.5 sec, about 0.4 sec, about 0.3 sec, and passes through the paddle, markers, breast, etc., and is received at the detector, where the x-ray energy is detected, operation 804. From this x-ray energy, an x-ray image may be generated, operation 806. The x-ray image includes at least the imaged breast and the markers. In the image, the marker includes an imaged characteristic. The image is analyzed in operation 808 to detect a movement of the marker. In examples, this analysis may include comparing the imaged marker characteristic to a physical marker characteristic, which may be obtained from a database in the imaging system. This database may include information about the paddle being utilized, physical characteristics of the rigid substrate and foam compressive element, as well as physical characteristics of the markers. After analysis, a signal may be sent in operation 810. This signal may be an emitted audible or displayed visual notification signal directed toward the technologist, and may indicate if a deviation between the imaged characteristic and the physical characteristic exceeds or is below a threshold. Such a signal may indicate to the technologist that the resulting image is or is not of a diagnostically-relevant quality. The method 800 may continue with processing the image of the breast and the marker to obtain an analysis image, operation 812. This analysis image is characterized by an absence of the marker. which may be removed by known image processing techniques, and is thus more appropriate for review and analysis by a radiologist. This analysis image may be displayed, operation 814.

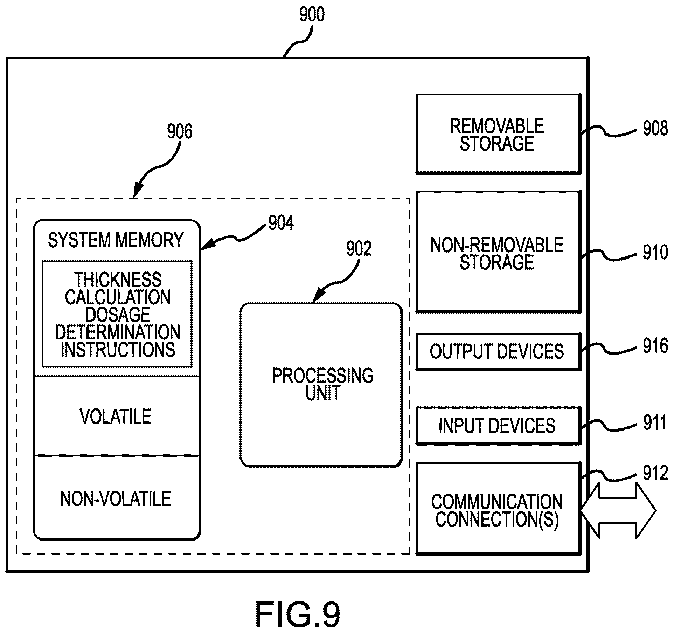

FIG. 9 illustrates one example of a suitable operating environment 900 in which one or more of the present examples can be implemented. This operating environment may be incorporated directly into the imaging systems disclosed herein, or may be incorporated into a computer system discrete from, but used to control, a the imaging and compression systems described herein. This is only one example of a suitable operating environment and is not intended to suggest any limitation as to the scope of use or functionality. Other well-known computing systems, environments, and/or configurations that can be suitable for use include, but are not limited to, imaging systems, personal computers, server computers, hand-held or laptop devices, multiprocessor systems, microprocessor-based systems, programmable consumer electronics such as smart phones, network PCs, minicomputers, mainframe computers, tablets, distributed computing environments that include any of the above systems or devices, and the like.

In its most basic configuration, operating environment 900 typically includes at least one processing unit 902 and memory 904. Depending on the exact configuration and type of computing device, memory 904 (storing, among other things, instructions to identify marker characteristics, determine x-ray dosages, compare imaged marker characteristics to known physical marker characteristics, or perform other methods disclosed herein) can be volatile (such as RAM), non-volatile (such as ROM, flash memory, etc.), or some combination of the two. This most basic configuration is illustrated in FIG. 9 by dashed line 906. Further, environment 900 can also include storage devices (removable, 908, and/or non-removable, 910) including, but not limited to, magnetic or optical disks or tape. Similarly, environment 900 can also have input device(s) 914 such as touch screens, keyboard, mouse, pen, voice input, etc., and/or output device(s) 916 such as a display, speakers, printer, etc. Also included in the environment can be one or more communication connections 912, such as LAN, WAN, point to point, Bluetooth, RF, etc.

Operating environment 900 typically includes at least some form of computer readable media. Computer readable media can be any available media that can be accessed by processing unit 902 or other devices having the operating environment. By way of example, and not limitation, computer readable media can include computer storage media and communication media. Computer storage media includes volatile and nonvolatile, removable and non-removable media implemented in any method or technology for storage of information such as computer readable instructions, data structures, program modules or other data. Computer storage media includes, RAM, ROM, EEPROM, flash memory or other memory technology, CD-ROM, digital versatile disks (DVD) or other optical storage, magnetic cassettes, magnetic tape, magnetic disk storage or other magnetic storage devices, solid state storage, or any other tangible medium which can be used to store the desired information. Communication media embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared and other wireless media. Combinations of the any of the above should also be included within the scope of computer readable media. A computer-readable device is a hardware device incorporating computer storage media.

The operating environment 900 can be a single computer operating in a networked environment using logical connections to one or more remote computers. The remote computer can be a personal computer, a server, a router, a network PC, a peer device or other common network node, and typically includes many or all of the elements described above as well as others not so mentioned. The logical connections can include any method supported by available communications media. Such networking environments are commonplace in offices, enterprise-wide computer networks, intranets and the Internet.

In some embodiments, the components described herein include such modules or instructions executable by computer system 900 that can be stored on computer storage medium and other tangible mediums and transmitted in communication media. Computer storage media includes volatile and non-volatile, removable and non-removable media implemented in any method or technology for storage of information such as computer readable instructions, data structures, program modules, or other data. Combinations of any of the above should also be included within the scope of readable media. In some embodiments, computer system 900 is part of a network that stores data in remote storage media for use by the computer system 900.

In embodiments, the various systems and methods disclosed herein may be performed by one or more server devices. For example, in one embodiment, a single server, such as server 904 may be employed to perform the systems and methods disclosed herein, such as the methods for imaging discussed herein. Client device 902 may interact with server 904 via network 908. In further embodiments, the client device 902 may also perform functionality disclosed herein, such as scanning and image processing, which can then be provided to servers 904 and/or 906.

Illustrative examples of the systems and methods described herein are provided below. An embodiment of the system or method described herein may include any one or more, and any combination of, the clauses described below:
Clause 1. A method of imaging a breast compressed with a paddle comprising a foam compressive element, the method comprising: emitting an x-ray energy from an x-ray source towards the breast and the foam compressive element and a marker disposed adjacent the foam compressive element, wherein the x-ray energy is emitted over a predetermined time period, and wherein the marker comprises a physical characteristic: detecting the x-ray energy at a detector disposed opposite the breast from the x-ray source: generating an image of the compressed breast and the marker based on the detected x-ray energy, wherein the marker in the generated image comprises an image characteristic associated with the physical characteristic; comparing the image characteristic to the physical characteristic; and sending a signal when the image characteristic deviates from the physical characteristic by a threshold.
Clause 2. The method of clause 1, wherein the physical characteristic comprises a physical dimension, and wherein the image characteristic depicts a movement of the marker during the emission of the x-ray energy.
Clause 3. The method of clause 2, wherein the physical dimension comprises a marker width and wherein the marker width is greater than a width of a pixel on the detector.
Clause 4. The method of any of clauses 1-3, wherein the marker is disposed on a surface of the foam compressive element that contacts the breast during the emission of the x-ray energy.
Clause 5. The method of any of clauses 1-4, wherein the marker is adhered to the breast.
Clause 6. The method of any of clauses 2-5, wherein the movement of the marker results from a movement of a portion of the breast during the emission of the x-ray energy.
Clause 7. The method of clause 6, wherein the portion of the breast is proximate the marker.
Clause 8. The method of any of clauses 1-7, further comprising: processing the image of the breast and the marker to generate an analysis image; and displaying the analysis image, wherein the marker is not visible in the analysis image.
Clause 9. The method of any of clauses 1-8, wherein the physical characteristic comprises a radiopacity, and wherein the image characteristic corresponds to an attenuation of the x-ray energy passing through the marker during the emission of the x-ray energy.
Clause 10. The method of any of clauses 2-9, wherein the marker comprises a marker width and wherein the marker width is greater than a width of a pixel on the detector.
Clause 11. The method of any of clauses 1-10, wherein sending the signal comprises displaying a notification.
Clause 12. An apparatus comprising: an x-ray source: an x-ray detector: a breast support platform for supporting a breast during an imaging procedure: a breast compression paddle movably disposed between the x-ray source and the x-ray detector, the breast compression paddle comprising: a rigid substrate: a foam compressive element secured to the rigid substrate; and a marker disposed in contact with the foam compressive element; at least one processor: and a memory storing instructions that, when executed by the at least one processor, cause the apparatus to perform operations comprising: emitting an x-ray energy from the x-ray source and though the breast and the marker; receiving the emitted x-ray energy: generating an image from the received x-ray energy, wherein the image depicts the breast and the marker; analyzing the image to identify a movement of the marker; and sending a signal when the movement exceeds a threshold.
Clause 13. The apparatus of clause 12, wherein analyzing the image comprises identifying the marker in the image.
Clause 14. The apparatus of clause 13, wherein the marker comprises a physical characteristic and wherein analyzing the image comprises comparing the physical characteristic of the marker to an imaged characteristic of the marker in the image.
Clause 15. The apparatus of clause 14, wherein the physical characteristic comprises a dimension of the marker.
Clause 16. The apparatus of any of clauses 14-15, wherein the physical characteristic comprises a radiopacity of the marker.
Clause 17. The apparatus of any of clauses 12-16, wherein the operations further comprise: processing the image of the breast and the marker to generate an analysis image: and displaying the analysis image, wherein the marker is not visible in the analysis image.
Clause 18. The apparatus of any of clauses 12-17, wherein sending the signal comprises displaying a notification.
Clause 19. The apparatus of any of clauses 12-18, wherein the marker is disposed on a surface of the foam compressive element that is configured to contact the breast during the emission of the x-ray energy.
Clause 20. The apparatus of any of clauses 12-19, wherein the breast compression paddle comprises the marker.
Clause 21. The apparatus of any of clauses 12-20, wherein the marker is disposed on a sheet adhered to the breast.
Clause 22. The apparatus of any of clauses 12-21, wherein the marker comprises a plurality of markers arranged in at least one row.
Clause 23. The apparatus of clause 22, wherein the at least one row comprises a plurality of rows.
Clause 24. The apparatus of any of clauses 22-23, wherein the row is disposed substantially parallel to a chest wall of the patient.
Clause 25. The apparatus of any of clauses 22-24, wherein the row is disposed substantially orthogonal to a chest wall of the patient.
Clause 26. The apparatus of any of clauses 23-25, wherein a first row of the plurality of rows is disposed substantially orthogonal to a second row of the plurality of rows.

This disclosure described some examples of the present technology with reference to the accompanying drawings, in which only some of the possible examples were shown. Other aspects can, however, be embodied in many different forms and should not be construed as limited to the examples set forth herein. Rather, these examples were provided so that this disclosure was thorough and complete and fully conveyed the scope of the possible examples to those skilled in the art.

Although specific examples were described herein, the scope of the technology is not limited to those specific examples. One skilled in the art will recognize other examples or improvements that are within the scope of the present technology. Therefore, the specific structure, acts, or media are disclosed only as illustrative examples. Examples according to the technology may also combine elements or components of those that are disclosed in general but not expressly exemplified in combination, unless otherwise stated herein. The scope of the technology is defined by the following claims and any equivalents therein.

## Claims

1. A method of imaging a breast compressed with a paddle, the method comprising:
emitting an x-ray energy from an x-ray source towards the breast and the paddle and a marker disposed adjacent the paddle, wherein the x-ray energy is emitted over a predetermined time period, and wherein the marker comprises a physical characteristic;
detecting the x-ray energy at a detector disposed opposite the breast from the x-ray source;
generating an image of the compressed breast and the marker based on the detected x-ray energy, wherein the marker in the generated image comprises an image characteristic associated with the physical characteristic;
comparing the image characteristic to the physical characteristic; and sending a signal when the image characteristic deviates from the physical characteristic by a threshold.

2. The method of claim 1, wherein the physical characteristic comprises a physical dimension, and wherein the image characteristic depicts a movement of the marker during the emission of the x-ray energy.

3. The method of claim 2, wherein the physical dimension comprises a marker width and wherein the marker width is greater than a width of a pixel on the detector.

4. The method of any of claims 1-3, wherein the marker is disposed on a surface of the paddle that contacts the breast during the emission of the x-ray energy.

5. The method of any of claims 1-4, wherein the marker is adhered to the breast.

6. The method of any of claims 2-5, wherein the movement of the marker results from a movement of a portion of the breast during the emission of the x-ray energy.

7. The method of claim 6, wherein the portion of the breast is proximate the marker.

8. The method of any of claims 1-7, further comprising:
processing the image of the breast and the marker to generate an analysis image; and
displaying the analysis image, wherein the marker is not visible in the analysis image.

9. The method of any of claims 1-8, wherein the physical characteristic comprises a radiopacity, and wherein the image characteristic corresponds to an attenuation of the x-ray energy passing through the marker during the emission of the x-ray energy.

10. The method of any of claims 2-9, wherein the marker comprises a marker width and wherein the marker width is greater than a width of a pixel on the detector.

11. The method of any of claims 1-10, wherein sending the signal comprises displaying a notification.

12. An apparatus comprising:
an x-ray source;
an x-ray detector;
a breast support platform for supporting a breast during an imaging procedure;
a breast compression paddle movably disposed between the x-ray source and the x-ray detector, the breast compression paddle comprising:
a marker disposed in contact with the paddle;
at least one processor; and
a memory storing instructions that, when executed by the at least one processor, cause the apparatus to perform operations comprising:
emitting an x-ray energy from the x-ray source and though the breast and the marker;
receiving the emitted x-ray energy;
generating an image from the received x-ray energy, wherein the image depicts the breast and the marker;
analyzing the image to identify a movement of the marker; and
sending a signal when the movement exceeds a threshold.

13. The apparatus of claim 12, wherein analyzing the image comprises identifying the marker in the image.

14. The apparatus of claim 13, wherein the marker comprises a physical characteristic and wherein analyzing the image comprises comparing the physical characteristic of the marker to an imaged characteristic of the marker in the image.

15. The apparatus of claim 14, wherein the physical characteristic comprises a dimension of the marker.

16. The apparatus of any of claims 14-15, wherein the physical characteristic comprises a radiopacity of the marker.

17. The apparatus of any of claims 12-16, wherein the operations further comprise:
processing the image of the breast and the marker to generate an analysis image; and
displaying the analysis image, wherein the marker is not visible in the analysis image.

18. The apparatus of any of claims 12-17, wherein sending the signal comprises displaying a notification.

19. The apparatus of any of claims 12-18, wherein the marker is disposed on a surface of the paddle that is configured to contact the breast during the emission of the x-ray energy.

20. The apparatus of any of claims 12-19, wherein the breast compression paddle comprises the marker.

21. The apparatus of any of claims 12-20, wherein the marker is disposed on a sheet adhered to the breast.

22. The apparatus of any of claims 12-21, wherein the marker comprises a plurality of markers arranged in at least one row.

23. The apparatus of claim 22, wherein the at least one row comprises a plurality of rows.

24. The apparatus of any of claims 22-23, wherein the row is disposed substantially parallel to a chest wall of the patient.

25. The apparatus of any of claims 22-24, wherein the row is disposed substantially orthogonal to a chest wall of the patient.

26. The apparatus of any of claims 23-25, wherein a first row of the plurality of rows is disposed substantially orthogonal to a second row of the plurality of rows.

27. The method of any of claims 1-11, or the apparatus of any of claims 12-26, wherein the paddle comprises a flexible feature, such as a plastic sheet, a flexible mesh or a foam compressive element, secured at or near a bottom surface of a rigid substrate.

28. The method or apparatus of claim 27, wherein the marker is disposed in contact with the flexible feature.

29. The method or apparatus of claim 28, wherein the marker is disposed on a surface of the flexible feature that is configured to contact the breast during the emission of the x-ray energy.

30. The method of any of claims 1-11, or the apparatus of any of claim 12-26, wherein the paddle comprises a rigid substrate and the marker is disposed on a surface of the rigid substrate.
